# EUROPEAN PATENT APPLICATION

(11) **EP 2 432 233 A2**
(43) Date of publication of application: **21.03.2012**
(21) Application number: 11160744.6
(22) Date of filing: 31.03.2011
(51) Int. Cl.: H04N 13/00

(54) **Display apparatus and image processing method thereof**

(30) Priority: 20.09.2010 KR 20100092261
(71) Applicant: SAMSUNG ELECTRONICS CO., LTD., Suwon-si Gyeonggi-do 442-742 (KR)
(72) Inventor: Hong, Sang-min, Suwon-si (KR)
(74) Representative: Waddington, Richard

(57) **Abstract**

A display apparatus and an image processing method thereof. The display apparatus including an image processing unit processing a left-eye image and a right-eye image; a display unit displaying the left-eye image and the right-eye image alternately; and a control unit controlling the image processing unit so that an image depth between the left-eye image and the right-eye image can be adjusted according to a vision difference between a user's two eyes.

## Description

### BACKGROUND

### 1. Field of the Invention

Apparatuses and methods consistent with exemplary embodiments relate to a display apparatus and an image processing method thereof, and more particularly, to a display apparatus displaying stereoscopic images and an image processing method thereof.

### 2. Description of the Related Art

With advances in technologies, display apparatuses process and display video signals in various forms, irrespective of whether the signals are of analog or digital. Recently, users have been viewing stereoscopic images, which refer to three-dimensional (3D) image signals, through monitors or televisions (TVs) and the like, allowing the users to easily access thereto. Different from two-dimensional (2D) image signals, stereoscopic image signals require that content be divided for left and right eyes of a user, and the divided images be respectively displayed for the user's left and right eyes.

For viewing stereoscopic images using the images respectively divided for the left and right eyes of the user, shutter glasses are provided to allow left and right scenes to be displayed alternately by switching on and off the shutter glasses.

When an image for the left eye and an image for the right eye are alternately displayed as described above, the user may feel dizziness or eye fatigue while viewing the stereoscopic images. The dizziness or fatigue may increase as a depth between the left-eye and right-eye images ("image depth") becomes larger.

### SUMMARY

Accordingly, one or more exemplary embodiments provide a display apparatus displaying stereoscopic images having different image depths according to vision differences and an image processing method thereof.

Another exemplary embodiment is to provide a display apparatus being capable of testing vision and an image processing method thereof.

Still another exemplary embodiment is to provide a display apparatus being capable of informing a user of a vision difference before and after viewing stereoscopic images.

According to the present invention there is provided an apparatus and method as set forth in the appended claims. Other features of the invention will be apparent from the dependent claims, and the description which follows.

The display apparatus according to one exemplary embodiment includes an image processing unit processing a left-eye image and a right-eye image, a display unit displaying the left-eye image and the right-eye image alternately, and a control unit controlling the image processing unit so that an image depth between the left-eye image and the right-eye image can be adjusted according to a vision difference between the left eye and the right eye of a user.

The control unit may control the image processing unit in such a manner that the image depth decreases as the vision difference between the left eye and the right eye increases.

The control unit may test vision of the two eyes based on a received user input.

The display apparatus may further include a user interface (UI) generating unit generating a vision testing UI for testing the user's vision and displaying it on the display unit.

The display apparatus may further include a distance measuring unit measuring a distance between the display unit and the user, and the control unit may control the UI generating unit so that the size of the vision testing UI is adjusted according to the measured distance.

The display apparatus may further include a synchronous signal output unit outputting a synchronous signal to open and close shutters of shutter glasses, comprising a left-eye shutter and a right-eye shutter, and the control unit may control the synchronous signal output unit so that the left-eye shutter and the right-eye shutter are alternately opened and closed while the user is testing vision of his/her eyes.

The control unit may control the UI generating unit in such a manner that at least two visions are generated as UI information, among the visions sequentially measured with a predetermined time difference.

The image display method of a display apparatus alternately displaying a left-eye image and a right-eye image, according to another exemplary embodiment, includes testing vision of a user's two eyes; adjusting an image depth between the left-eye image and the right-eye image according to th e measured vision difference; and displaying the left-eye image and the right-eye image between which the image depth is adjusted.

The display apparatus according to a still another exemplary embodiment includes a display unit displaying a vision testing UI testing visions thereon, shutter glasses opening a left-eye shutter while a vision on the left eye is tested, and opening a right-eye shutter while a vision on the right eye is tested, and a synchronous signal output unit outputting a synchronous signal to open and close the shutters to the shutter glasses.

The foregoing and/or other aspects may be achieved by providing a display apparatus displaying stereoscopic images having different image depth according to vision differences, and an image processing method thereof.

The foregoing and/or other aspects may also be achieved by providing a display apparatus being capable of testing a user's vision, and an image processing method thereof.

The foregoing and/or other aspects may also be achieved by providing a display apparatus being capable of informing the user of a vision difference before and after viewing the stereoscopic images.

### BRIEF DESCRIPTION OF THE DRAWINGS

The above and/or other aspects will become apparent and mo r e readily appreciated from th e following description of the exemplary embodiments, taken in conjunction with the accompanying drawings, in which:
FIG. 1 is a control block diagram showing a display apparatus according to an exemplary embodiment;
FIG. 2 is a diagram showing an image for a left eye ("left-eye image") and an image for a right eye ("right-eye image") displayed on the display apparatus according to an exemplary embodiment;
FIG. 3 is a control block diagram showing a display apparatus of another exemplary embodiment;
FIG. 4 is a diagram showing a vision testing user interface (UI) displayed on the display apparatus according to another exemplary embodiment;
FIG. 5 is a control flow chart explaining about an image display method of a display apparatus according to another exemplary embodiment;
FIG. 6 is a control flow chart explaining about an image display method of a display apparatus according to a still another exemplary embodiment;
FIG. 7 is a diagram showing vision test values of the display apparatus according to a still another exemplary embodiment;
FIG. 8 is a schematic diagram showing a display apparatus according to a still another exemplary embodiment; and
FIG. 9 is a diagram showing an alarm message displayed on the display apparatus according to a still another exemplary embodiment.

### DETAILED DESCRIPTION OF EXEMPLARY EMBODIMENTS

Below, exemplary embodiments will be described in detail with reference to accompanying drawings so as to be easily realized by a person having ordinary knowledge in the art. The exemplary embodiments may be embodied in various forms without being limited to the exemplary embodiments set forth herein. Descriptions of well-known parts are omitted for clarity, and like reference numerals refer to like elements throughout.

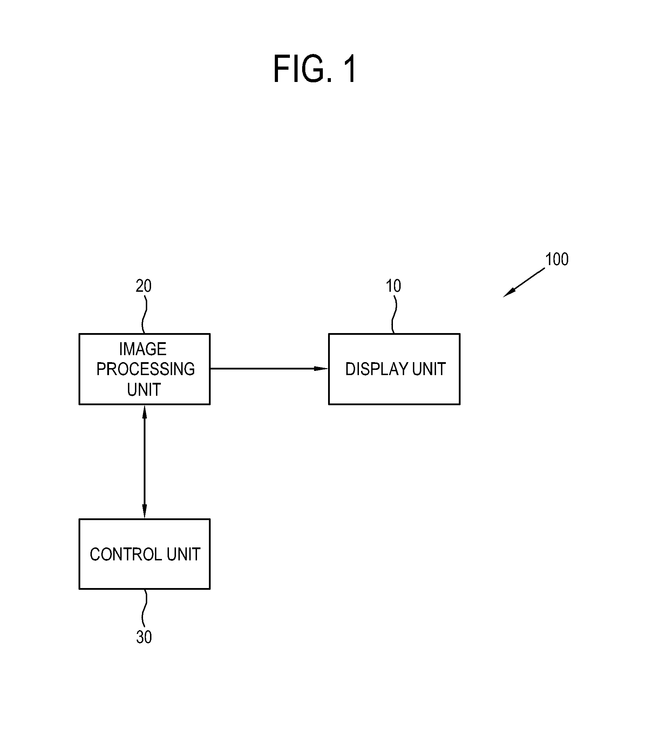

FIG. 1 is a control block diagram showing a display apparatus 100 according to an exemplary embodiment.

As shown therein, the display apparatus 100 includes a display unit 10, an image processing unit 20, and a control unit 30 which controls the display unit 10 and the image processing unit 20. The display apparatus 100 of the present exemplary embodiment may be embodied by a television that processes and displays broadcasting signals, a monitor connected to a computer main body, a portable terminal such as mobile phones, or the like. The display apparatus 100 can receive and display two-dimensional (2D) image signals (namely plane image signals) or three-dimensional (3D) image signals (namely stereoscopic image signals). A stereoscopic image includes an image to be viewed through the user's left eye ("left-eye image") and an image to be viewed through the user's right eye ("right-eye image"), and the left-eye and right-eye images are alternately displayed on a frame basis. The display apparatus 100 may further include a signal receiving unit (not shown) receiving an image signal from an image source.

The display unit 10 displays a stereoscopic image formed with a right-eye image and a left-eye image. The display unit 10 may include a liquid crystal panel having a liquid crystal layer or an organic light emitting panel having a light emitting layer formed of an organic matter, and further includes a panel driving unit driving them.

The image processing unit 20 divides the received 3D image signal into a right-eye image and a left-eye image, and processes them so that the right-eye image and the left-eye image are alternately displayed on the display unit 10 according to a certain frame rate. The image processing unit 10 may also process an input 2D image signal to generate a right-eye image and a left-eye image.

The stereoscopic image may have its stereoscopic sense adjusted by a difference between the right-eye image and the left-eye image, called an image depth. As the image depth is larger, the stereoscopic sense felt by the user increases, thereby making an object viewed to be more projected or more depressed. As the image depth is smaller, the stereoscopic sense felt by the user decreases. The image processing unit 20 of the present exemplary embodiment adjusts the image depth between the right-eye image and the left-eye image, as controlled by the control unit 30.

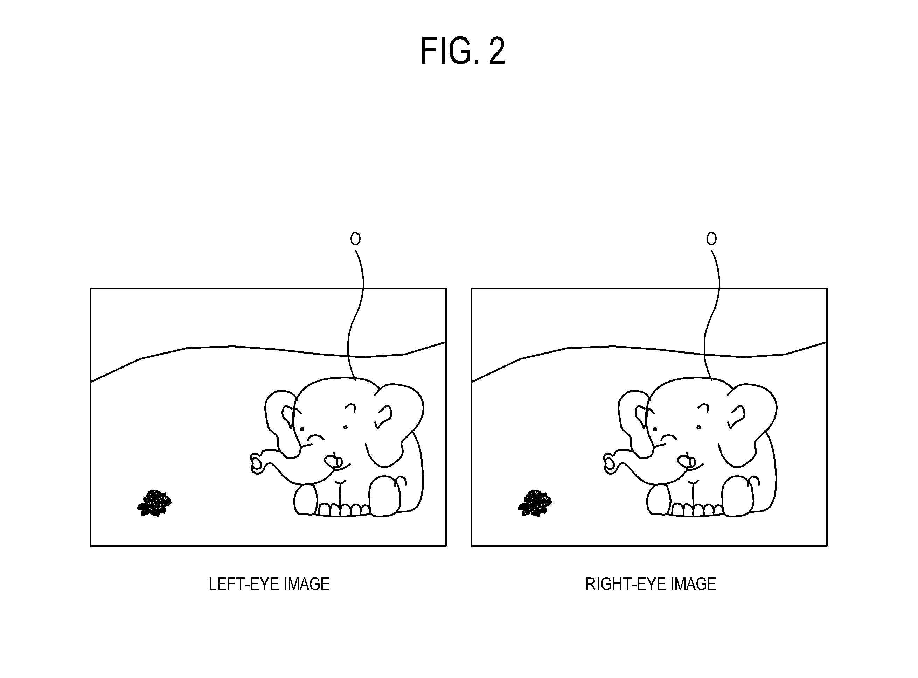

The control unit 30 controls the image processing unit 20 so as to allow the image depth between the right-eye image and the left-eye image to be adjusted according to a difference in vision between the two eyes of the user ("vision difference"). FIG. 2 shows a right-eye image and a left-eye image displayed on the display apparatus 100 according to the present exemplary embodiment. As shown therein, the right-eye image and the left-eye image constituting one frame are different from each other. They are identical in background images, constituting a scene, but positions of the objects requiring a three dimensional effect are different on the right-eye image and on the left-eye image. This difference in image forms an image depth, and the stereoscopic sense increases as the image depth is larger.

This stereoscopic image conveys a liveliness effect, rendering realism, to the user, but it may also cause the user to feel dizziness or increase a fatigue in vision. This problem may become worse when the vision difference between the two eyes of the user is larger. Accordingly, the control unit 30 controls the image processing unit 20 to have the image depth decrease as the vision difference between the two eyes of the user increases. The control unit 30 may receive information about the user's vision from the outside, or the user may input his/her vision information through a user input unit (not shown).

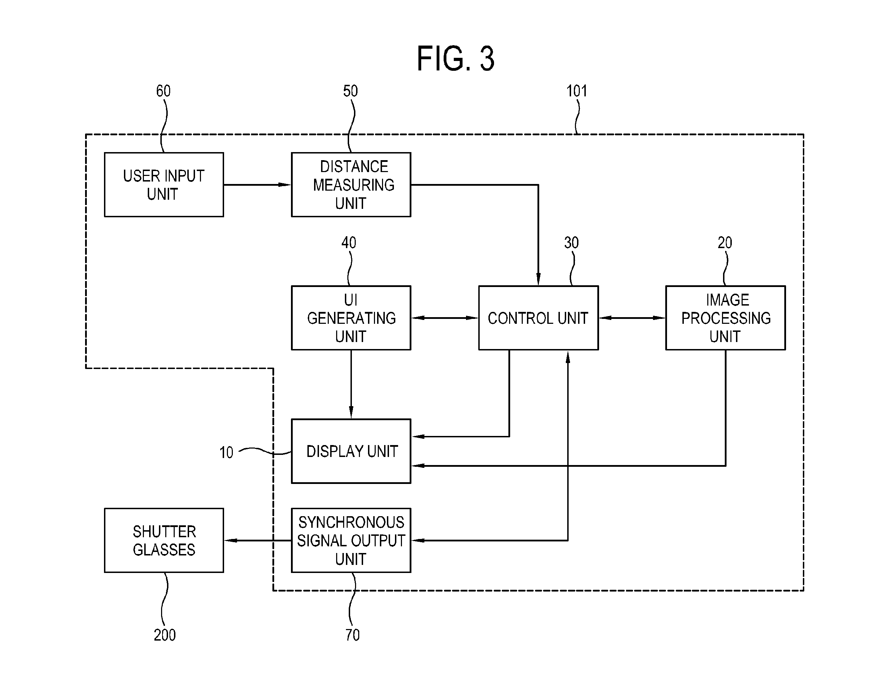

FIG. 3 is a control block diagram showing a display apparatus 101 according to another exemplary embodiment.

The display apparatus 101 according to the present exemplary embodiment may further include a UI generating unit 40, a distance measuring unit 50, a user input unit 60 and a synchronous signal outputting unit 70, in addition to the configurations shown in FIG. 1. The display apparatus 101 outputs a synchronous signal to the shutter glasses 200 so that opening and closing of the shutter glasses 200 can be controlled.

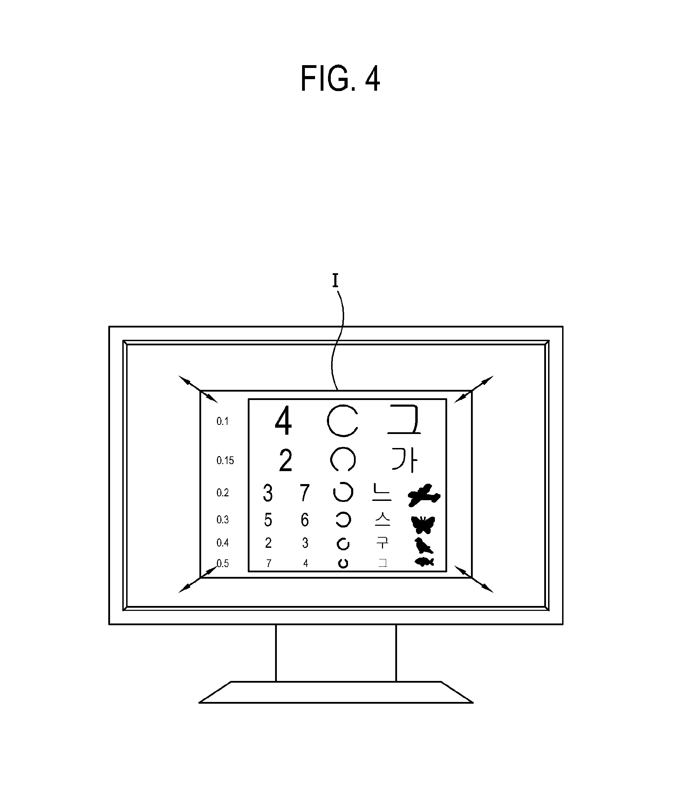

The UI generating unit 40 generates UI information and outputs it to the display unit 10. The UI generating unit 40, according to the present exemplary embodiment, generates a vision testing UI for testing a user's vision according to control by the control unit 30. FIG. 4 shows the vision testing UI (I) according to the present exemplary embodiment. The vision testing UI (I) includes numerals, diagrams, figures and so on, which vary in size, and may further include characters in public domain for the vision test. The vision testing UI (I) may also include a pointer or a cursor, which can indicate any character or can be moved on the character.

For accurate test of the user's vision, it is necessary to make the vision testing UI (I) separate from the user by a certain distance. However, to provide for a case that the certain distance is not maintained, it is desirable that the vision testing UI (I) is adjusted in size depending upon the distance between the user and the display part 10. The distance measuring unit 50 measures a distance between the display unit 10 and the user, and the control unit 30 controls the UI generating unit 40 so that the size of the vision testing UI (I) is adjusted based on the measured distance. For example, if the distance between the display unit 10 and the user increases, the size of the vision testing UI (I) increases. Whereas if the distance between the display unit 10 and the user decreases, the size of the vision test UI (I) decreases. The distance measuring unit 50 may be embodied by an image taking apparatus or an optical apparatus being capable of measuring the distance by use of an infrared ray, a radio frequency (RF) or laser.

The user input unit 60 corresponds to a user interface for input by a user. The user can input whether a character displayed on the vision testing UI (I) is visible or not, by use of the user input unit 60. The user input unit 60 may include direction buttons, numeral buttons or selection buttons and so on, or may further include a wheel-type input unit or a touch pad. The user input unit 60 may be embodied by a remote control, being capable of outputting an input signal remotely. The user may also be able to check characters visible by his/her own eye by moving the pointer or the cursor, with the use of the selection buttons or the touch pad. In addition, the user may adjust the size of the vision testing UI (I), or select a vision testing UI that the user prefers, from a plurality of vision testing UIs.

The control unit 30 measures vision of the two eyes based on the received user input and outputs information about a vision difference between the two eyes to the image processing unit 20. The control unit 30 may include information about the vision testing UI (I), and data having a lookup table, allowing the user's vision to be determined, based on the information.

The synchronous signal output unit 70 generates a synchronous signal to alternately open the right-eye shutter (not shown) and the left-eye shutter (not shown) of the shutter-type glasses 200, and transmits it to the shutter glasses 200. The synchronous signal output unit 70 may include an infrared ray transmitting unit. If the intensity of the infrared signal transmitted as the synchronous signal is beyond a certain threshold value, either of the right-eye shutter or the left-eye shutter is opened; if the intensity of the infrared signal is less than the certain threshold value, the other shutter is opened. The synchronous signal output unit 70 may be embodied by a wire/wireless interface to communicate with the shutter glasses 200. The synchronous signal output unit 70 according to the present exemplary embodiment allows the right-eye shutter and the left-eye shutter to be alternately opened while testing the user's vision according to control by the control unit 30. When the left-eye shutter is opened, the vision testing UI (I) is visible only through the user's left-eye, whereas when the right-eye shutter is opened, the vision testing UI (I) is visible only through the user's right-eye.

The shutter glasses 200 alternatively open and close the right-eye shutter and the left-eye shutter, triggered by the synchronous signal output from the synchronous signal output unit 70. The shutter glasses 200 may include a liquid crystal shutter.

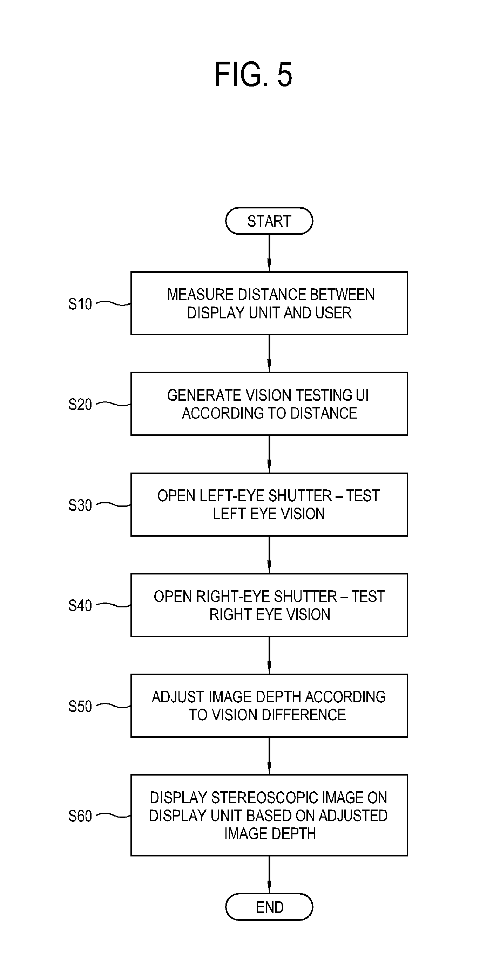

FIG. 5 is a control flow chart explaining an image display method of a display apparatus according to the present exemplary embodiment. Referring to FIG. 5, the control method of the present exemplary embodiment for adjusting an image depth according to the vision test will be described below.

The distance measuring unit 50 first measures a distance between the display unit 10 and the user (S10).

Then, the control unit 30 controls the UI generating unit 40 to generate a vision testing UI (I) whose size is adjusted according to the measured distance, and displays the vision testing UI (I) on the display unit 10 (S20).

The synchronous signal output unit 70 outputs a synchronous signal to alternately open and close the right-eye shutter and the left-eye shutter, to the shutter glasses 200 having the right-eye shutter and the left-eye shutter, and the user tests his/her vision for the right-eye and left-eye respectively (S30 and S40).

The control unit 30 receives a user input based on the vision testing UI (I) and determines the vision of the two eyes, and controls the image processing unit 20 to adjust an image depth between the right-eye image and the left-eye image, based on a difference between the measured vision (S50).

The image processing unit 20 decreases the image depth as the vision difference between the two eyes increases, but it increases the image depth as the vision difference between the two eyes decreases, to thereby adjust the image depth, and displays a stereoscopic image on the display unit 10 according to the adjusted image depth (S60).

According to another exemplary embodiment, the user may input the vision of each of the two eyes on the display apparatus 101, rather than measuring his/her vision through the vision testing UI (I). In this case, the UI generating unit 40 may display an input window (not shown) to allow the user to directly input his/her vision on the display unit 10, and the control unit 30 may control the image processing unit 20 so as to allow the image depth to be adjusted according to the vision difference.

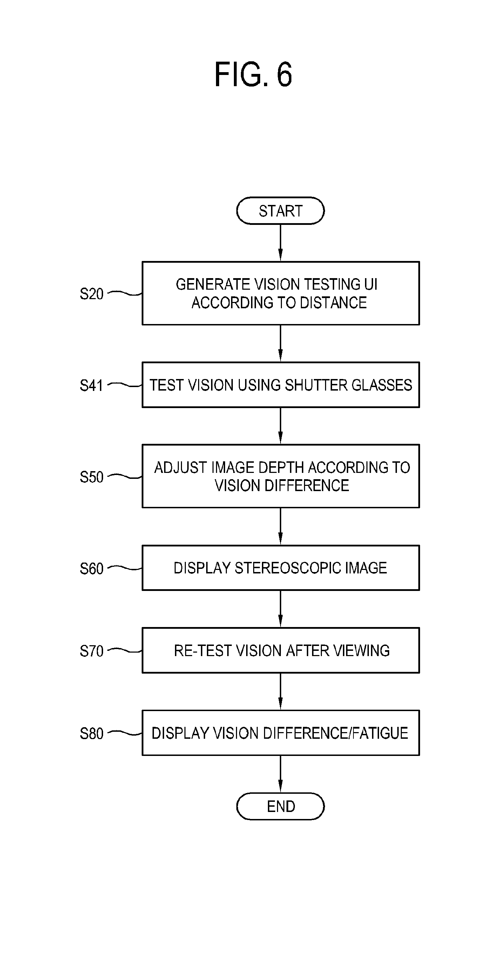

FIG. 6 is a control flow chart explaining an image display method of a display apparatus according to another exemplary embodiment. As illustrated therein, the display apparatus according to the present exemplary embodiment displays the vision testing UI (I) according to a distance between the display unit and the user as shown in FIG. 5 (S20), and tests the vision of the two eyes using the shutter glasses 200 (S41).

The display apparatus adjusts the image depth depending upon the vision difference between the two eyes (S50) and displays a stereoscopic image on the display unit 10 based on the adjusted image depth (S60).

When the user stops viewing the stereoscopic image, the control unit 30, according to the present exemplary embodiment, controls the UI generating unit 40 so that the user can re-test his/her vision (S70).

Thereafter, the control unit 30 displays at least one of the measured vision difference and fatigue on the display unit 10 (S80). In other words, the control unit 30 controls the UI generating unit 40 so as to generate at least two visions as UI information, among a plurality of visions sequentially measured with a predetermined time difference. The user can determine to what degree he/she has a fatigue in his/her eyes based on the UI information, and can adjust his/her habit of viewing the stereoscopic images.

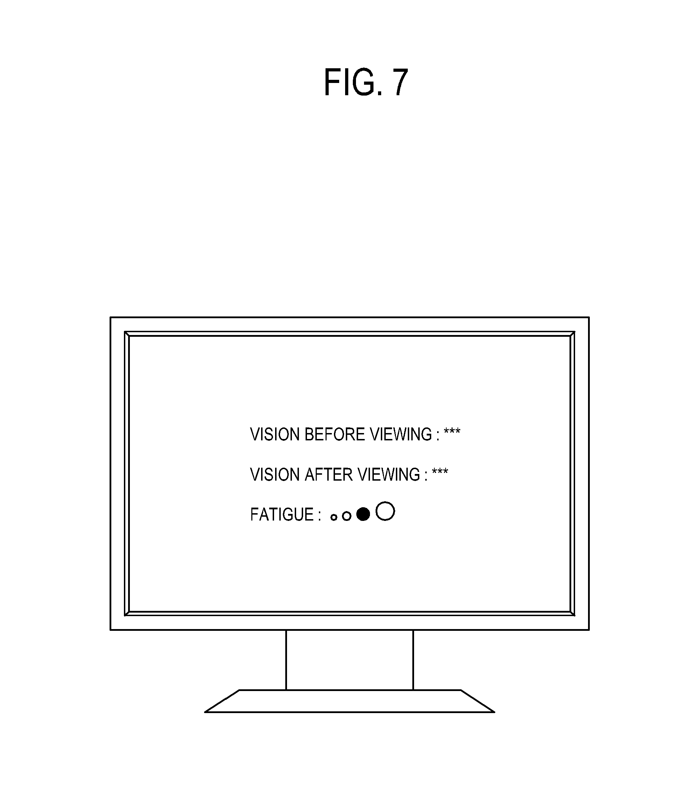

FIG. 7 is a diagram showing vision test values of the display apparatus according to a another exemplary embodiment. On the display unit 10 are displayed vision values measured before and after viewing the stereoscopic images and the user's fatigue after viewing them. It is determined that the higher the vision difference is, the higher the fatigue is. The fatigue-associated UI can display the degree of fatigue depending upon the vision difference, including figures and numerals, etc. It is preferably displayed in graphic so as to allow the user to readily recognize it.

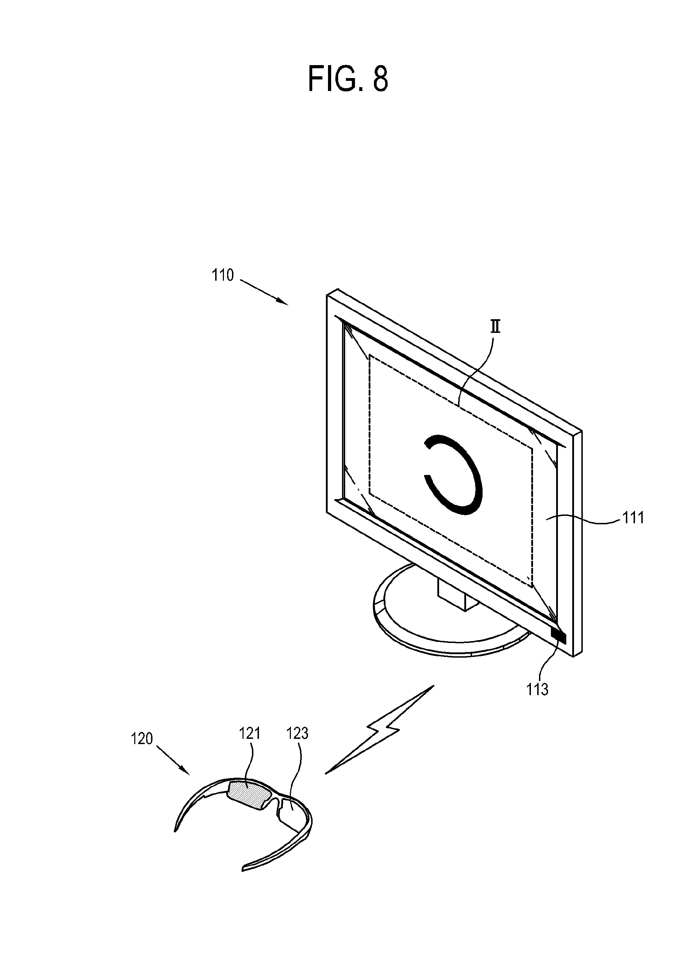

FIG. 8 is a schematic diagram showing a display apparatus according to a another exemplary embodiment. As shown therein, the display apparatus according to the present exemplary embodiment includes a display main body 110 and a pair of shutter glasses 120 that are opened and closed according to a synchronous signal output from the display main body 110. The display main body 110 includes a display unit 111 and a synchronous signal output unit 113.

The display unit 111 displays a vision testing UI (II) for measuring the user's vision. The display unit 111 may include a UI generating unit for generating the vision testing UI (II) and a storage unit. If a control signal such as a user input is received, the display unit 111 displays the vision testing UI (II), allowing the user to test the vision of his/her right and left eyes. The vision testing UI (II) may include diverse characters, numerals, figures, etc.

The synchronous signal output unit 113 outputs a synchronous signal to open and close the shutters of the shutter glasses 120.

The shutter glasses 120 include a left-eye shutter 121 and a right-eye shutter 123. When a vision for the user's left eye is tested, the left-eye shutter 121 is opened according to the synchronous signal and the right-eye shutter 123 is closed, and vice versa. That is, when a vision for the user's right eye is tested, the right-eye shutter 123 is opened and the left-eye shutter 121 is automatically closed.

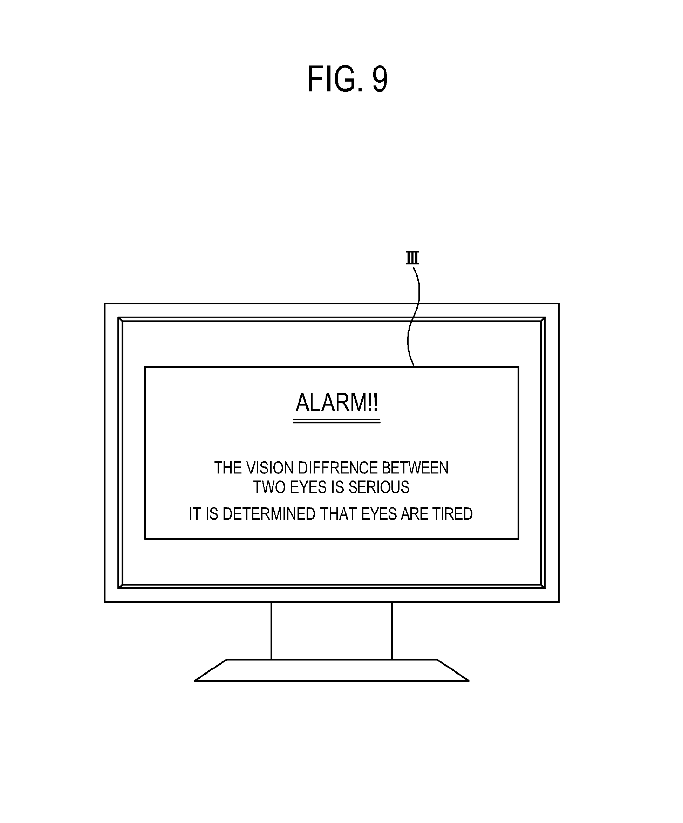

FIG. 9 is a diagram showing an alarm message displayed on the display apparatus according to a still another exemplary embodiment.

According to this exemplary embodiment, the display unit 111 displays an alarm message (III) where a vision difference between the left eye and the right eye exceeds a predetermined threshold value. The alarm message (III) may indicate that the vision difference between the two eyes is serious or how much the eyes are tired. The alarm message may also indicate a recommendation to stop viewing a television or viewing the stereoscopic images.

The present invention is characterized in that the image depth can be adjusted according to the vision difference between the user's two eyes in the display apparatus displaying stereoscopic images, which is liable for causing the user to feel dizziness or fatigue on the eyes.

Although a few exemplary embodiments have been shown and described, it will be appreciated by those skilled in the art that changes may be made in these exemplary embodiments without departing from the principles of the invention, the scope of which is defined in the appended claims and their equivalents.

Attention is directed to all papers and documents which are filed concurrently with or previous to this specification in connection with this application and which are open to public inspection with this specification, and the contents of all such papers and documents are incorporated herein by reference.

All of the features disclosed in this specification (including an y accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in th i s specification (including any accompanying claims, abstract and drawings), or to any novel one, or any novel combination, of the steps of any method or process so disclosed.

## Claims

1. A display apparatus, comprising:
an image processing unit that processes a left-eye image and a right-eye image;
a display unit that displays the left-eye image and the right-eye image alternately; and
a control unit that controls the image processing unit so that an image depth between the left-eye image and the right-eye image is adjusted according to a vision difference between a left eye and a right eye of a user.

2. The display apparatus according to claim 1, wherein the control unit controls the image processing unit in such a manner that the image depth decreases as the vision difference between the left eye and the right eye of the user increases.

3. The display apparatus according to claim 1 or claim 2, wherein the control unit tests vision of the left eye and the right eye of the user based on a received user input.

4. The display apparatus according to any preceding claim, further comprising a user interface (UI) generating unit that generates a vision testing UI that tests the user's vision in each of the left eye and the right eye and displays the vision testing UI on the display unit.

5. The display apparatus according to claim 4, further comprising a distance measuring unit that measures a distance between the display unit and the user,
wherein the control unit controls the UI generating unit so that the size of the vision testing UI is adjusted according to the measured distance.

6. The display apparatus according to any preceding claim, further comprising a synchronous signal output unit outputting a synchronous signal to open and close shutters of shutter glasses, comprising a left-eye shutter and a right-eye shutter,
wherein the control unit controls the synchronous signal output unit so that the left-eye shutter and the right-eye shutter are alternately opened and closed while the user is testing vision of the left eye and the right eye.

7. The display apparatus according to claim 4, wherein the control unit controls the UI generating unit in such a manner that at least two visions are generated as UI information, among the visions sequentially measured with a predetermined time difference.

8. An image display method of a display apparatus alternately displaying a left-eye image and a right-eye image, comprising:
testing vision of a left eye and vision of a right eye of a user;
adjusting an image depth between the left-eye image and the right-eye image according to the measured vision difference between the left eye and the right eye;
displaying the left-eye image and the right-eye image to which the image depth is adjusted.

9. The image display method according to claim 8, wherein the controlling of the image depth comprises decreasing the image depth as the vision difference between the left eye and the right eye increases.

10. The image display method according to claim 8 or claim 9, wherein the testing of the vision of the left eye and the vision of the right eye comprises:
displaying a vision testing user interface (UI) that tests the vision of the left eye and the vision of the right eye; and
receiving a user input based on the vision testing UI and determining the vision of the left eye and the vision of the right eye.

11. The image display apparatus according to claim 10, wherein the displaying of the vision testing UI comprises:
measuring a distance between a display unit and the user; and
adjusting the size of the vision testing UI according to the measured distance.

12. The image display method according to any one of claims 8 to 11, wherein the testing of the vision of the left eye and the vision of the right eye comprises alternately opening and closing a left-eye shutter and a right-eye shutter using shutter glasses comprising the left-eye shutter and the right-eye shutter.

13. The image display method according to any one of claims 8 to 12, further comprising:
measuring the vision of the left eye and the vision of the right eye sequentially with a predetermined time difference; and
displaying at least two measured visions as UI information
